# EUROPÄISCHE PATENTSCHRIFT

(11) **EP 1 351 945 B1**
(45) Veröffentlichungstag und Bekanntmachung des Hinweises auf die Patenterteilung: **23.01.2008**
(21) Anmeldenummer: 01965269.2
(22) Anmeldetag: 10.09.2001
(51) Int. Cl.: C07D 277/28, A61Q 5/10

(54) **NEUE 1,4-DIAMINO-2-(THIAZOL-2-YL)-BENZOL-DERIVATE UND DIESE VERBINDUNGEN ENTHALTENDE FÄRBEMITTEL**
NOVEL 1,4-DIAMINO-2-(THIAZOL-2-YL)BENZENE DERIVATIVES AND DYES CONTAINING SAID COMPOUNDS
NOUVEAUX DERIVES DE 1,4-DIAMINO-2-(THIAZOL-2-YL)-BENZENE ET COLORANTS CONTENANT LESDITS COMPOSES

(30) Priorität: 18.01.2001 DE 10102084
(43) Veröffentlichungstag der Anmeldung: 15.10.2003
(73) Patentinhaber: Wella Aktiengesellschaft, 64274 Darmstadt (DE)
(72) Erfinder: CHASSOT, Laurent, 1724 Praroman (CH); BRAUN, Hans-Jürgen, 3182 Überstorf (CH)
(86) Internationale Anmeldenummer: PCT/EP2001/010407
(87) Internationale Veröffentlichungsnummer: WO 2002/057243

(56) Entgegenhaltungen:
- EP-A- 1 052 252
- DE-A- 2 125 193
- DE-C- 19 812 059
- DE-U- 20 108 704
- US-A- 4 828 568

## Beschreibung

Die Erfindung betrifft neue 1,4-Diamino-2-(thiazol-2-yl)-benzol-Derivate sowie diese Verbindungen enthaltende Mittel zum Färben von Keratinfasern.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol 1,4-Diaminobenzol und 4,5-Diaminopyrazol eingesetzt, während als Kupplersubstanzen beispielsweise Resorcin, 4-Chlorresorcin, 1-Naphthol, 3-Aminophenol, m-Phenylendiamin, 2-Amino-4-(2'-hydroxyethyl)amino-anisol, 1,3-Diamino-4-(2'-hydroxyethoxy)benzol und 2,4-Diamino-5-fluor-toluol zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann.

In der DE-PS 198 12 059 und der DE-GM 299 12 882 wird empfohlen, anstelle der seit langem bekannten p-Phenylendiamine neue 2,5-Diamino-1-(2-thienyl)-benzole als Entwicklersubstanzen in Oxidationsfärbemittein zu verwenden.

Es bestand jedoch weiterhin ein Bedürfnis nach neuen Entwickler substanzen, welche die an Oxidationsfarbstoffvorstufen gestellten Anforderungen in besonderem Masse erfüllen.

Hierzu wurde nun überraschenderweise gefunden, daß neue 1,4-Diamino-2-(thiazol-2-yl)-benzol-Derivate gemäß der allgemeinen Formel (1) die an Entwicklersubstanzen gestellten Anforderungen in besonders hohem Masse erfüllen. So werden unter Verwendung dieser Entwicklersubstanzen mit den meisten bekannten Kupplersubstanzen farbintensive. Farbnuancen erhalten, die außerordentlich lichtecht und waschecht sind.

Gegenstand der vorliegende Erfindung sind daher 1,4-Diamino-2-(thiazol-2-yl)-benzol-Derivate der allgemeinen Formel (I) oder deren physiologisch verträgliche, wasserlösliche Salze, 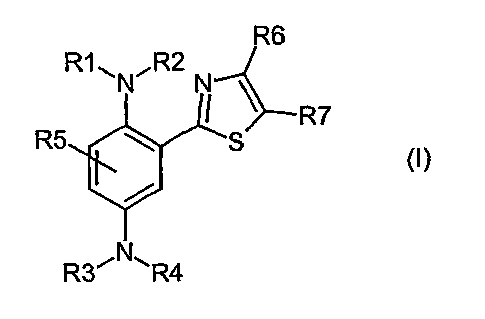 worin
**R1,R2,R3 und R4** unabhängig voneinander Wasserstoff, eine C₁-C₆-Alkylgruppe, eine C₂-C₄-Hydroxyalkylgruppe, eine C₃-C₄ Dihydroxyalkylgruppe oder eine C₂-C₄-Alkoxy-(C₁-C₂)-alkylgruppe darstellen oder R1und R2 beziehungsweise R3 und R4 einen viergliedrigen bis achtgliedrigen aliphatischen Ring bilden, wobei mindestens 2 der Reste R1 bis R4 Wasserstoff darstellen;
**R5** gleich Wasserstoff, einem Halogenatom (F, Cl, Br, J), einer C₁-C₄-Alkylgruppe, einer C₁-C₄ -Hydroxyalkylgruppe oder einer C₁-C₄-Alkoxygruppe ist; **R6 und R7** unabhänging voneinander gleich Wasserstoff, einem Halogenatom (F, Cl, Br, J), einer Nitrogruppe, einer C₁-C₄ -Alkoxygruppe, einer C₁-C₆. Alkylgruppe, einer C₁-C₄-Hydroxyalkylgruppe, einer C₃-C₄-Dihydroxyalkylgruppe oder einer einem Rest der Formel (II) sind 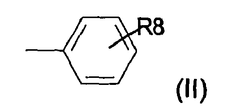 **R8** gleich Wasserstoff, einer Aminogruppe einem Halogenatom (F, Cl, Br, J), einer Nitrogruppe oder einer Hydroxygruppe ist.

Als geeignete Verbindungen der Formel (1) können beispielweise die folgenden Verbindungen genannt werden:
1,4-Diamino-2-(thiazol-2-yl)-benzol; 1,4-Diamino-2-(4-methyl-thiazoll-2-yl)-benzol; 1,4-Diamino-2-(5-methyl-thiazol-2-yl)-benzol; 1,4-Diamino-2-(4-chlor-thiazol-2-yl)-benzol; 1,4-Diamino-2-(5-chlor-thiazol-2-yl)-benzol; 1,4-Diamino-2-(5-nitro-thiazol-2-yl)-benzol; 1,4-Diamino-2-(4,5-dimethylthiazol-2-yl)-benzol; 1,4-Diamino-2-(4-methyl-5-(2-hydroxyethyl)-thiazol-2-yl)-benzol 1,4-Diamino-2-(4-phenyl-thiazol-2-yl)-benzol; 1,4-Diamino-2-(5-nitro-thiazol-2-yl)-benzol ;1,4-Diamino-2-(5-phenyl-thiazol-2-yl)-benzol; 1-Amino-4-methylamino-2-(thiazol-2-yl)-benzol; 1-Amino-4-(2-hydroxyethyl)amino-2-(thiazol-2-yl)-benzol und 1-Amino-4-bis(2-hydroxyethyl)-amino-2-(thiazol-2-yl)-benzol.

Bevorzugt sind Verbindungen der Formel (1), bei denen (i) eine oder mehrere der Restgruppen R5, R6 und R7 gleich Wasserstoff sind und/oder (ii) R1, R2, R3 und R4 gleichzeitig Wasserstoff bedeuten und/oder (iii) eine oder beide Restgruppen R6 und R7 gleich C₁-C₄-Alkyl oder C₁-C₄-Hydroxyalkyl sind.

Insbesondere sind die folgenden Verbindungen zu nennen:
1,4-Diamino-2-(thiazol-2-yl)-benzol; 1,4-Diamino-2-(4,5-dimethyl-thiazol-2-yl)-benzol; 1,4-Diamino-2-(4-phenyl-thiazol-2-yl)-benzol und 1,4-Diamino-2-(4-methyl-thiazol-2-yl)-benzol.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die Herstellung der erfindungsgemäßen 1,4-Diamino-2-(thiazol-2-yl)-benzol-Derivate der Formel (1) kann unter Verwendung von bekannten Syntheseverfahren erfolgen. Die Synthese der erfindungsgemäßen Verbindungen kann beispielsweise wie folgt durchgeführt werden:
Entweder a) durch eine Tetrakis(triphenylphospin)palladium(0) katalysierte Kupplung eines substituierten Benzols der Formel (III) 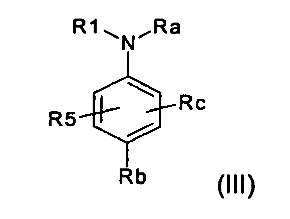 mit einer Heteroarylverbindung der Formel (IV) 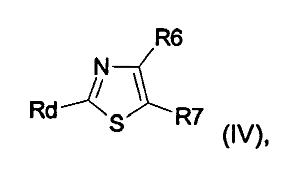 worin
   Ra die Bedeutung einer Schutzgruppe, wie sie zum Beispiel in dem Kapitel "Protective Groups" in Organic Synthesis, Kapitel 7, Wiley Interscience, 1991 beschrieben wird,
   Rb die Bedeutung NR1 Ra oder NO₂ hat,
   Rc die Bedeutung Halogen und Rd die Bedeutung B(OH)₂ beziehungsweise Rc die Bedeutung B(OH)₂ und Rd die Bedeutung Halogen hat, und
   R1, R5, R6 und R7 die in Formel (1) gennante Bedeutung haben, und anschließende Abspaltung der Schutzgruppe oder Abspaltung der Schutzgruppe und Reduktion der Nitrogruppe;
oder b) durch eine Tetrakis(triphenylphospin)palldium(0) katalysierte Kupplung eines substituierten Benzols der Formel (V) 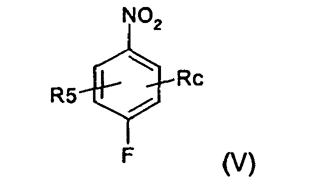 mit einer Heteroarylverbindung der Formel (IV) 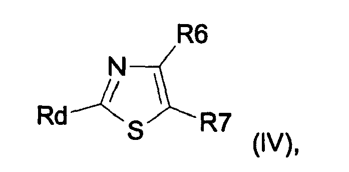 worin
   Rc die Bedeutung Halogen und Rd die Bedeutung B(OH)₂ beziehungsweise Rc die Bedeutung B(OH)₂ und Rd die Bedeutung Halogen hat, und
   R5, R6 und R7 die in Formel (1) angegebene Bedeutung haben, anschließende Substitution des so erhaltenen substituierten Benzols der Formel (VI) 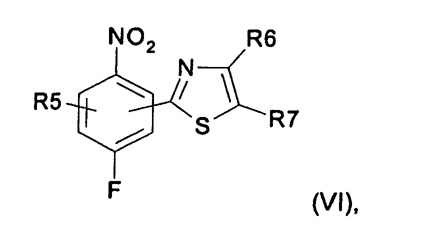 mit einem Amin der Formel HNR1 R2,
   worin R1,R2 die in Formel (I) genannte Bedeutung haben, und
   abschliesende Reduktion der Nitrogruppe.

Die erfindungsgemäßen 1,4-Diamino-2-(thiazol-2-yl)-benzol-Derivate der Formel (I) sind in Wasser gut löslich und ermöglichen Färbungen mit hoher Farbintensität und ausgezeichneter Farbechtheit, insbesondere was die Lichtechtheit, Waschechtheit und Reibeechtheit anbetrifft. Die Verbindungen der Formen (I) weisen weiterhin eine ausgezeichnete Lagerstabilität, insbesondere als Bestandteil der nachfolgend beschriebenen Färbemittel, auf.

Ein weiterer Gegenstand der vorliegenden Erfindung sind daher Mittel zum oxidativen Färben von Keratinfasern, wie zum Beispiel Haaren, Pelzen, Federn oder Wolle, insbesondere menschlichen Haaren, auf der Basis einer Entwicklersubstanz-Kupplersubstanz-Kombination, welche als Entwicklersubstanz mindestens ein 1,4-Diamino-2-(thiazol-2-yl)-benzol-Derivat der Formel (I) enthalten.

Das 1,4-Diamino-2-(thiazol-2-yl)-benzol-Derivat der Formel (I) ist in dem erfindungsgemäßen Färbemittel in einer Menge von etwa 0,005 bis 20 Gewichtsprozent enthalten, wobei eine Menge von etwa 0,01 bis 5,0 Gewichtsprozent und insbesondere 0,1 bis 2,5 Gewichtsprozent bevorzugt ist.

Als Kupplersubstanzen kommen vorzugsweise N-(3-Dimethylaminophenyl)-harnstoff, 2,6-Diamino-pyridin, 2-Amino-4-[(2-hydroxyethyl)-amino]-anisol, 2,4-Diamino-1-fluor 5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxy ethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxy-propoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion in Betracht.

Obwohl die vorteilhaften Eigenschaften der hier beschriebenen 1,4-Diamino-2-(thiazol-2-yl)-benzol-Derivate der Formel (1) es nahelegen, diese als alleinige Entwicklersubstanz zu verwenden, ist es selbstverständlich auch möglich, die 1,4-Diamino-2-(thiazol-2-yl)-benzol-Derivate der Formel (I) gemeinsam mit bekannten Entwicklersubstanzen, wie zum Beispiel 1,4-Diaminobenzol, 2,5-Diaminotoluol, 2,5-Diaminophenylethylalkohol, 4-Aminophenol und seinen Derivaten, beispielsweise 4-Amino-3-methylphenol, 4,5-Diamino-1-(2-hydroxyethyl)-pyrazol, 4,5-Diamino-1-benzyl-pyrazol, 4,5-Diamino-1-(4-methyl-benzyl)-pyrazol oder Tetraaminopyrimidinen, einzusetzen.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6,0 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Uberschuß oder Unterschuß vorhanden sind.

Weiterhin kann das erfindungsgemäße Färbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe wie 4-[(4'-aminophenyl)-(4'imino-2",5"-cyclohexadien-1 "-yliden)-methyl]-2-methylaminobenzol-monohydrochlorid (C.l. 42 510) und 4-[(4'amino-3'-methyl-phenyl)-(4"-imino-3"-methyl-2",5"cyclohexadien-1"-yliden)-methyl]-2-methyl-aminobenzol monohydrochlorid (C.l. 42 520), aromatische Nitrofarbstoffe wie 4-(2'-hydroxyethyl)-amino-nitrotoluol, 2-Amino-4,6-dinitrophenol, 2-Amino-5-(2'-hydroxyethyl)-amino-nitrobenzol, 2-Chlor-6-(ethylamino)-4-nitrophenol, 4-Chlor-N-(2-hydroxyethyl)-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5hydroxy-naphthalin-1-sulfonsäure-Natriumsalz (C.1. 14 805) und Dispersionsfarbstoffe wie beispielsweise 1,4-Diaminoanthrachinon und 1,4,5,8-Tetraaminoantrachinon, enthalten. Die Färbemittel können diese Farbkomponenten in einer Menge von etwa 0,1 bis 4,0 Gewichtsprozent enthalten.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein. Die Zubereitungsform des erfindungsgemäßen Färbemittels kann beispielsweise eine Lösung, insbesondere eine wäßrige oder wäßrigalkoholische Lösung sein. Die besonders bevorzugten Zubereitungsformen sind jedoch eine Creme, ein Gel oder eine Emulsion. Ihre Zusammensetzung stellt eine Mischung der Farbstofflcomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die erfindungsgemäßen Färbemittel mit einem Gehalt an 1,4-Diamino-2-(thiazol-2-yl)-benzol-Derivaten der Formel (I) als Entwicklersubstanz ermöglichen Färbungen von Keratinfasern, insbesondere Haaren, mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Färbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus. Die sehr guten färberischen Eigenschaften der Färbemittel gemäß der vorliegenden Anmeldung zeigen sich weiterhin darin, dass diese Mittel eine Anfärbung von ergrauten, chemisch nicht vorgeschädigten Haaren problemlos und mit guter Deckkraft ermöglichen.

Die nachfolgenden Beispiele sollen den Gegenstand der Erfindung näher erläutern, ohne ihn darauf zu beschränken.

### Beispiele

### Beispiel 1: Synthese von 1,4-Diamino-2-(thiazol-2-yl)-benzol

### A. Synthese von N,N'-Bis(tert-Butoxycarbonyl)-2,5-diamino-1-phenylborsäure

Die N,N'-Bis(tert-Butoxycarbonyl)-2,5-diamino-1-phenylborsäure wird durch Umsetzung von N,N'-Bis(tert-Butoxycarbonyl)-2,5-diamino-1-brombenzol mit tert-Butyllithium und Trimethylborate dargestellt. Die experimentelle Vorschrift dieser Herstellungsmethode wird von J. M. Tour und J. J. S. Lamba in J. Am. Chem. Soc.1994, 116, Seite 11723 beschrieben.

### B. Synthese von 1,4-Diamino-2-(thiazol-2-yl)-benzol Hydrochlorid

0,035 g (0,0001 mol) 2,5-tert.-Butyloxycarbonylamino-1-phenylborsäure aus Stufe A und 0,00015 mol 2-Brom-thiazol werden unter Argon in 10 ml 1,2-Dimethoxyethan gelöst. Anschliessend werden 0,005 g Tetrakis-(triphenylphosphin)-palladium (0,000005 mol) und 0,13 ml einer 2normalen Kaliumcarbonatlösung zugegeben und die Reaktionsmischung auf 80 °C erwärmt. Nach Beendigung der Reaktion wird die Reaktionsmischung in 10 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol auf 50 °C erwärmt. Anschliessend werden zur Herstellung des Hydrochlorides 1,5 ml einer 2,9molaren ethanolische Salzsäurelösung zugetropft. Der Niederschlag wird abfiltriert, zweimal mit 1 ml Ethanol gewaschen und sodann getrocknet.
Ausbeute: 0,015 g (94 % der Theorie)
Masspektrum: MH⁺ 192 (100)

### Beispiel 2: Haarfärbemittel

Es werden Haarfärbelösungen der folgenden Zusammensetzung hergestellt:

| | |
|---|---|
| 1,25 mmol | 1,4-Diamino-2-(thiazol-2-yl)-benzol*HCl |
| 1,25 mmol | Kupplersubstanz gemäß Tabelle 1 |
| 1,0 g | Kaliumoleat (8-prozentige wässrige Lösung) |
| 1,0 g | Ammoniak (22-prozentige wässrige Lösung) |
| 1,0 g | Ethanol |
| 0,3 g | Ascorbinsäure |
| ad 100,0 g | Wasser |

50 g der vorstehenden Färbelösung werden unmittelbar vor der Anwendung mit 50 g einer 6prozentigen wässrigen Wasserstoffperoxidlösung vermischt. Anschliessend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die erhaltenen Färbungen sind in Tabelle 1 zusammengefaßt.

**Tabelle 1:**

| **Kupplersubstanz** | **Farbe** |
|---|---|
| a) 1,3-Dihydroxy-benzol | dunkelblond |
| b) 1,3-Diamino-4-(2-hydroxy-ethoxy)-benzol-sulfat | dunkelblau |
| c) 5-Amino-2-methyl-phenol | purpur |
| d) 1-Naphtol | blau |

### Beispiele 3 bis 12: Haarfärbemittel

Es werden Haarfärbelösungen der folgenden Zusammensetzung hergestellt:

| | |
|---|---|
| X g | 1,4-Diamino-2-(thiazol-2-yl)-benzol*HCl (**E1**) |
| U g | Entwicklersubstanz **E8** bis **E15** gemäß Tabelle 2 |
| Y g | Kupplersubstanz **K11** bis **K36** gemäß Tabelle 4 |
| Z g | direktziehender Farbstoff **D1** bis **D3** gemäß Tabelle 3 |
| 10,0 g | Kaliumoleat (8-prozentige wässrige Lösung) |
| 10,0 g | Ammoniak (22-prozentige wässrige Lösung) |
| 10,0 g | Ethanol |
| 0,3 g | Ascorbinsäure |
| ad 100,0 g | Wasser |

30 g der vorstehenden Färbelösung werden unmittelbar vor der Anwendung mit 30 g einer 6prozentigen wässsrigen Wasserstoffperoxidlösung vermischt. Anschliessend wird das Gemisch auf gebleichte Haare aufgetragen. Nach einer Einwirkungszeit von 30 Minuten bei 40°C wird das Haar mit Wasser gespült, mit einem handelsüblichen Shampoo gewaschen und getrocknet. Die Färbeergebnisse sind in Tabelle 5 zusammengefasst.

### Beispiele 13 bis 18: Haarfärbemittel

Es werden cremeförmige Farbträgermassen der folgenden Zusammensetzung hergestellt:

| | |
|---|---|
| Xg | 1,4-Diamino-2-(thiazol-2-yl)-benzol*HCl (**E1**) |
| U g | Entwicklersubstanz **E8** bis **E15** gemäß Tabelle 2 |
| Y g | Kupplersubstanz **K11** bis **K36** gemäss Tabelle 4 |
| Z g | direktziehender Farbstoff **D2** gemäss Tabelle 3 |
| 15,0 g | Cetylalkohol |
| 0,3 g | Ascorbinsäure |
| 3,5 g | Natriumlaurylalkoholdiglycolethersulfat, 28-prozentige wässrige Lösung |
| 3,0 g | Ammoniak, 22-prozentige wässrige Lösung |
| 0,3 g | Natriumsulfit, wasserfrei |
| ad 100,0 g | Wasser |

30 g der vorstehenden Färbecreme werden unmittelbar vor der Anwendung mit 30 g einer 6-prozentigen Wasserstoffperoxidlösung vermischt. Anschliessend wird das Gemisch auf das Haar aufgetragen. Nach einer Einwirkzeit von 30 Minuten bei 40°C wird das Haar mit Wasser gespült, mit einem handelsüblichen Shampoo gewaschen und getrocknet. Die Färbeergebnisse sind in Tabelle 6 zusammengefasst.

**Tabelle 2:**

| | |
|---|---|
| **Entwicklersubstanzen** | |
| **E1** | 1,4-Diamino-2-(thiazol-2-yl)-benzol*HCl |
| | |
| **E8** | 1,4-Diaminobenzol |
| **E9** | 2,5-Diamino-phenylethanol-sulfat |
| **E10** | 3-Methyl-4-amino-phenol |
| **E11** | 4-Amino-2-aminomethyl-phenol-dihydrochlorid |
| **E12** | 4-Amino-phenol |
| **E13** | N,N-Bis(2'-hydroxyethyl)-p-phenylendiamin-sulfat |
| **E14** | 4,5-Diamino-1-(2'-hydroxyethyl)-pyrazol-sulfat |
| **E15** | 2,5-Diaminotoluol-sulfat |

**Tabelle 3:**

| **Direktziehende Farbstoffe** | |
|---|---|
| **D1** | 2,6-Diamino-3-((pyridin-3-yl)azo)pyridin |
| **D2** | 6-Chlor-2-ethylamino-4-nitro-phenol |
| **D3** | 2-Amino-6-chlor-4-nitro-phenol |

**Tabelle 4:**

| **Kupplersubstanzen** | |
|---|---|
| **K11** | 1,3-Diaminobenzol |
| **K12** | 2-Amino-4-(2'-hydroxyethyl)amino-anisol-sulfat |
| **K13** | 1,3-Diamino-4-(2'-hydroxyethoxy)benzol-sulfat |
| **K14** | 2,4-Diamino-5-fluor-toluol-sulfat |
| **K15** | 3-Amino-2-methylamino-6-methoxy-pyridin |
| **K16** | 3,5-Diamino-2,6-dimethoxy-pyridin-dihydrochlorid |
| **K17** | 2,4-Diamino-5-ethoxy-toluol-sulfat |
| **K18** | N-(3-Dimethylamino)phenylharnstoff |
| **K19** | 1,3-Bis(2,4-Diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K26** | 1-Acetoxy-2-methyl-naphthalin |
| **K31** | 1,3-Dihydroxy-benzol |
| **K32** | 2-Methyl-1,3-dihydroxy-benzol |
| **K33** | 1-Chlor-2,4-dihydroxy-benzol |
| **K34** | 4-(2'-Hydroxyethyl)amino-1,2-methylendioxybenzolhydrochlorid |
| **K35** | 3,4-Methylendioxy-phenol |
| **K36** | 2-Amino-5-methyl-phenol |

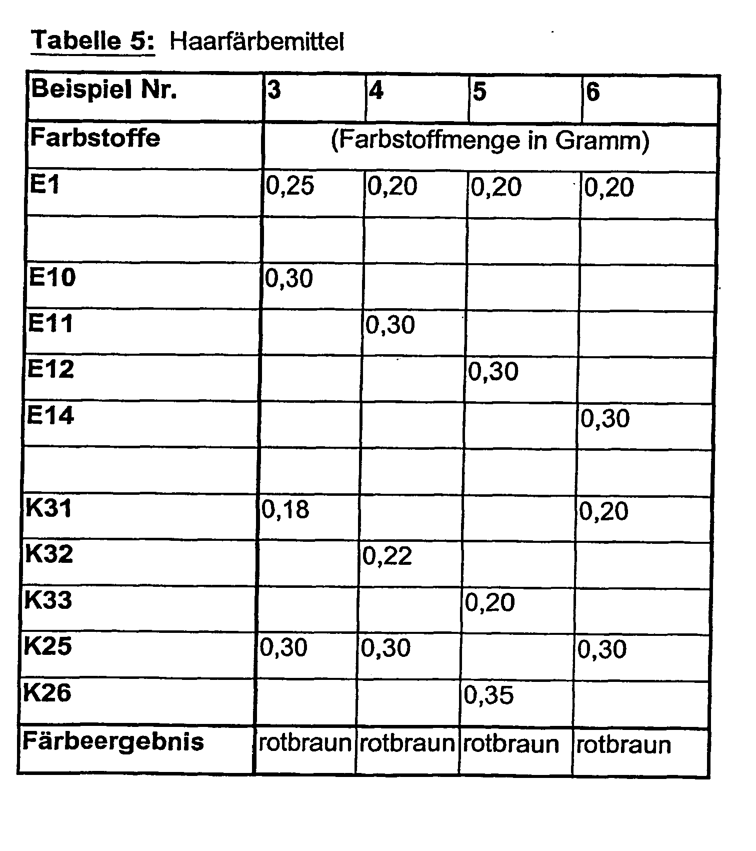

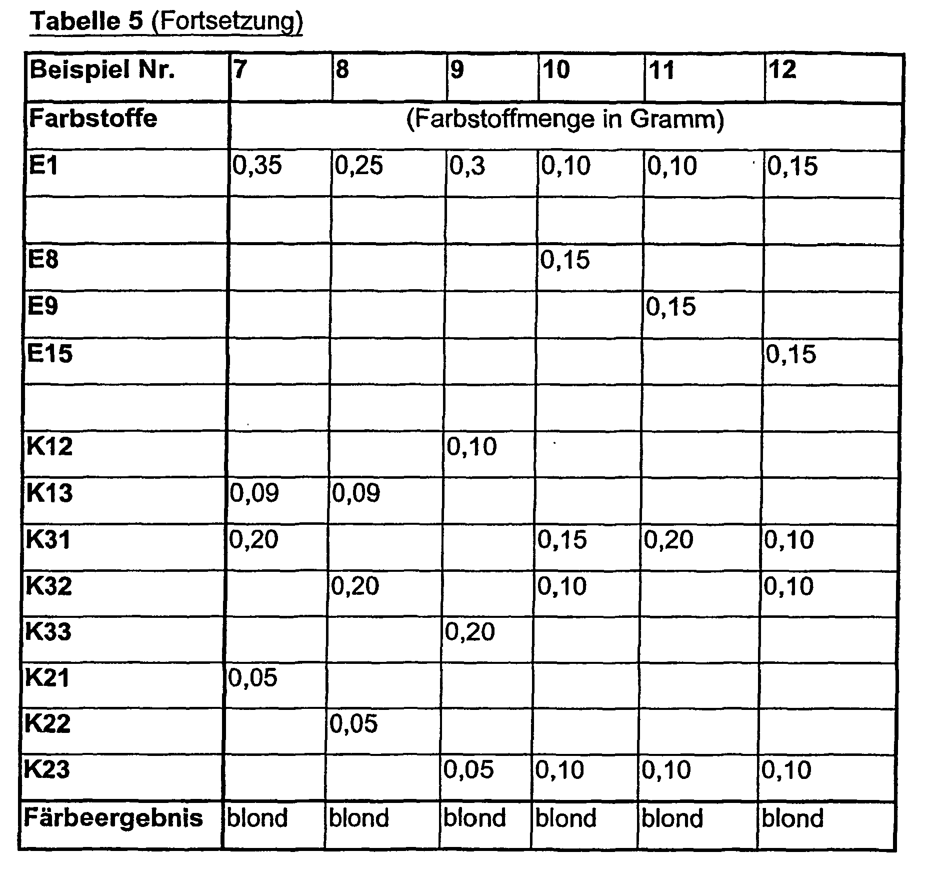

**Tabelle 6: Haarfärbemittel**

| **Beispiel Nr.** | **13** | **14** | **15** | **16** | **17** | **18** |
|---|---|---|---|---|---|---|
| **Farbstoffe** | (Farbstoffmenge in Gramm) | | | | | |
| **E1** | 1,80 | 1,80 | 1,80 | 0,70 | 0,70 | 0,70 |
| | | | | | | |
| **K12** | | | | 0,10 | 0,10 | 0,10 |
| **K13** | 1,10 | 1,10 | 1,10 | | | |
| **K31** | 1,10 | 1,10 | 1,10 | 0,40 | 0,40 | 0,40 |
| | | | | | | |
| **D2** | | | | 0,10 | 0,10 | 0,10 |
| **K23** | | | 0,05 | 0,10 | 0,10 | 0,10 |
| **Färbeergebnis** | schwarz | schwarz | schwarz | braun | braun | braun |

Alle in der vorliegenden Anmeldung enthaltenen Prozentangaben stellen soweit nicht anders angegeben Gewichtsprozente dar.

## Patentansprüche

1. 1,4-Diamino-2-(thiazol-2-yl)-benzol-Derivate der Formel (1) 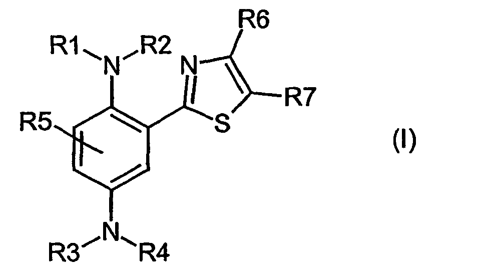 worin
**R1,R2,R3** und **R4** unabhängig voneinander Wasserstoff, eine C₁-C₆-Alkylgruppe, eine C₂-C₄-Hydroxyalkylgruppe, eine C₃-C₄ Dihydroxyalkylgruppe oder eine C₂-C₄-Alkoxy-(C₁-C₂)-alkylgruppe darstellen oder R1 und R2 beziehungsweise R3 und R4 einen viergliedrigen bis achtgliedrigen aliphatischen Ring bilden, wobei mindestens 2 der Reste R1 bis R4 Wasserstoff darstellen;
**R5** gleich Wasserstoff, einem Halogenatom, einer C₁-C₄-Alkylgruppe, einer C₁-C₄ -Hydroxyalkylgruppe oder einer C₁-C₄-Alkoxy-gruppe ist;
**R6 und R7** unabhänging voneinander gleich Wasserstoff, einem Halogenatom, einer Nitrogruppe, einer C₁-C₄ -Alkoxygruppe, einer C₁-C₆-Alkylgruppe, einer C₁-C₄-Hydroxyalkylgruppe, einer C₃-C₄-Dihydroxyalkylgruppe oder einer einem Rest der Formel (II) sind 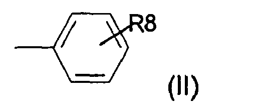 **R8** gleich Wasserstoff, einer Aminogruppe einem Halogenatom, einer Nitrogruppe oder einer Hydroxygruppe ist.

2. 1,4-Diamino-2-(thiazol-2-yl)-benzol-Derivat nach Anspruch 1, **dadurch gekennzeichnet, dass** in der Formel (I) einer oder mehrere der Reste R5 bis R7 gleich Wasserstoff sind.

3. 1,4-Diamino-2-(thiazol-2-yl)-benzol-Derivat nach Anspruch 1 oder 2, **dadurch gekennzeichnet, dass** in der Formel (1) die Reste R1, R2, R3 und R4 gleich Wasserstoff sind.

4. 1,4-Diamino-2-(thiazol-2-yl)-benzol-Derivat nach einem der Ansprüche 1 bis 3, **dadurch gekennzeichnet, dass** in der Formel (1) einer oder beide Reste R6 und R7 gleich einer C₁-C₄-Alkylgruppe oder einer C₁-C₄-Hydroxyalkylgruppe sind.

5. 1,4-Diamino-2-(thiazol-2-yl)-benzol-Derivat nach einem der Ansprüche 1 bis 4, **dadurch gekennzeichnet, dass** es ausgewählt ist aus 1,4-Diamino-2-(thiazol-2-yl)-benzol; 1,4-Diamino-2-(4,5-dimethyl-thiazot-2-yl)-benzol; 1,4-Diamino-2-(4-phenyl-thiazol-2-yl)-benzol und 1,4-Diamino-2-(4-methyl-thiazol-2-yl)-benzol.

6. Mittel zum oxidativen Färben von Keratinfasern, auf der Basis einer Entwicklersubstanz-Kupplersubstanz-Kombination, **dadurch gekennzeichnet, dass** es als Entwicklersubstanz mindestens ein 1,4-Diamino-2-(thiazol-2-yl)-benzol-Derivat der Formel (I) nach einem der Ansprüche 1 bis 5 enthält.

7. Mittel nach Anspruch 6, **dadurch gekennzeichnet, dass** es das 1,4-Diamino-2-(thiazol-2-yl)-benzol-Derivat der Formel (I) in einer Menge von 0,005 bis 20,0 Gewichtsprozent enthält.

8. Mittel nach Anspruch 6 oder 7, **dadurch gekennzeichnet, dass** die Kupplersubstanz ausgewählt ist aus 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anitin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

9. Mittel einem der Ansprüche 6 bis 8, **dadurch gekennzeichnet, dass** es zusätzlich zum 1,4-Diamino-2-(thiazol-2-yl)-benzol-Derivat der Formel (1) mindestens eine weitere Entwicklersubstanz aus der Gruppe bestehend aus 1,4-Diaminobenzol, 2,5-Diaminotoluol, 2-(2,5-Diamino-phenyl)-ethylalkohol, 4-Aminophenol und seinen Derivaten, 4,5-Diaminopyrazolderivaten und Tetraaminopyrimidinen enthält.

10. Mittel nach einem der Ansprüche 6 bis 9, **dadurch gekennzeichnet, dass** die Entwicklersubstanzen und Kupplersubstanzen, bezogen auf die Gesamtmenge des Oxidationsfärbemittel, jeweils in einer Gesamtmenge von 0,005 bis 20 Gewichtsprozent enthalten sind.

11. Mittel nach einem der Ansprüche 6 bis 10 , **dadurch gekennzeichnet, daß** es zusätzlich mindestens einen direktziehenden Farbstoff enthält.

12. Mittel nach einem der Ansprüche 6 bis 11, **dadurch gekennzeichnet, daß** es einen pH-Wert von 6,5 bis 11,5 aufweist.

13. Mittel nach einem der Ansprüche 6 bis 12, **dadurch gekennzeichnet, dass** es in Form einer wässrigen oder wässrigalkoholischen Lösung, einer Creme, eines Gels oder einer Emulsion vorliegt.

14. Mittel nach einem der Ansprüche 6 bis 13, **dadurch gekennzeichnet, dass** es ein Haarfärbemittel ist.

## Claims

1. 1,4-Diamino-2-(thiazol-2-yl)benzene derivatives of the formula (I) 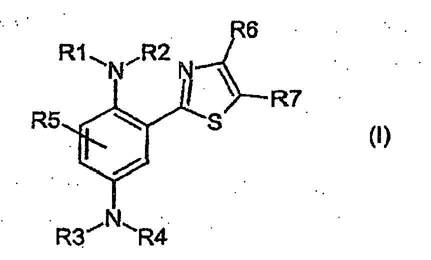 in which
**R1, R2, R3 and R4,** independently of one another, are hydrogen, a C₁-C₈-alkyl group, a C₂-C₄-hydroxyalkyl group, a C₃-C₄-dihydroxyalkyl group or a C₂-C₄-alkoxy-(C₁-C₂)-alkyl group, or R1 and R2, or R3 and R4, form a four-membered to eight-membered aliphatic ring, where at least 2 of the radicals R1 to R4 are hydrogen;
**R5** is hydrogen, a halogen atom, a C₁-C₄-alkyl group, a C₁-C₄-hydroxyalkyl group or a C₁-C₄-alkoxy group;
**R6 and R7,** independently of one another, are hydrogen, a halogen atom, a nitro group, a C₁-C₄-alkoxy group, a C₁-C₆-alkyl group, a C₁-C₄-hydroxyalkyl group, a C₃-C₄-dihydroxyalkyl group or a radical of the formula (II) 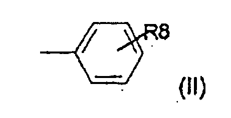 **R8** is hydrogen, an amino group, a halogen atom, a nitro group or a hydroxy group.

2. 1,4-Diamino-2-(thiazol-2-yl)benzene derivative according to Claim 1, **characterized in that**, in the formula (I), one or more of the radicals R5 to R7 are hydrogen.

3. 1,4-Diamino-2-(thiazol-2-yl)benzene derivative according to Claim 1 or 2, **characterized in that**, in the formula (I), the radicals R1, R2, R3 and R4 are hydrogen.

4. 1,4-Diamino-2-(thiazol-2-yl)benzene derivative according to one of Claims 1 to 3, **characterized in that**, in the formula (I), one of or both radicals R6 and R7 are a C₁-C₄-alkyl group or a C₁-C₄-hydroxyalkyl group.

5. 1,4-Diamino-2-(thiazol-2-yl)benzene derivative according to one of Claims 1 to 4, **characterized in that** it is selected from 1,4-diamino-2-(thiazol-2-yl)benzene; 1,4-diamino-2-(4,5-dimethylthiazol-2-yl)-benzene; 1,4-diamino-2-(4-phenylthiazol-2-yl)benzene and 1,4-diamino-2-(4-methylthiazol-2-yl)benzene.

6. Agent for the oxidative colouring of keratin fibres, based on a developer substance-coupler substance combination, **characterized in that** it comprises, as developer substance, at least one 1,4-diamino-2-(thiazol-2-yl)benzene derivative of the formula (I) according to one of Claims 1 to 5.

7. Agent according to Claim 6, **characterized in that** it comprises the 1,4-diamino-2-(thiazol-2-yl)benzene derivative of the formula (I) in an amount of from 0.005 to 20.0 per cent by weight.

8. Agent according to Claim 6 or 7, **characterized in that** the coupler substance is selected from 2,6-diaminopyridine, 2-amino-4-[(2-hydroxyethyl)amino]-anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxy-ethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxy-pyridine, 1,3-diaminobenzene, 2,4-diamino-1-(2-hydroxyethoxy)benzene, 2,4-diamino-1,5-di(2-hydroxyethoxy)-benzene, 1-(2-aminoethoxy)-2,4-diaminobenzene, 2-amino-1-(2-hydroxyethoxy)-4-methylaminobenzene, 2,4-diamino-phenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)-amino]aniline, 3-[(2-aminoethyl)amino]aniline, 1,3-di-(2,4-diaminophenoxy)propane, di(2,4-diaminophenoxy)-methane, 1,3-diamino-2,4-dimethoxybenzene, 2,6-bis-(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-2-methylphenol, 3-[(2-hydroxyethyl)amino]phenol, 3-[(2-methoxyethyl)amino]phenol, 5-amino-2-ethylphenol, 2-(4-amino-2-hydroxyphenoxy)ethanol, 5-[(3-hydroxypropyl)-amino]-2-methylphenol, 3-[(2,3-dihydroxypropyl)amino]-2-methylphenol, 3-[(2-hydroxyethyl)amino]-2-methylphenol, 2-amino-3-hydroxypyridine, 5-amino-4-chloro-2-methylphenol, 1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxol, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

9. Agent according to one of Claims 6 to 8, **characterized in that**, in addition to the 1,4-diamino-2-(thiazol-2-yl)benzene derivative of the formula (I), it comprises at least one further developer substance from the group consisting of 1,4-diaminobenzene, 2,5-diaminotoluene, 2-(2,5-diaminophenyl)ethyl alcohol, 4-aminophenol and its derivatives, 4,5-diaminopyrazole derivatives and tetraaminopyrimidines.

10. Agent according to one of Claims 6 to 9, **characterized in that** the developer substances and coupler substances, based on the total amount of the oxidation colourant, are present in each case in a total amount of from 0.005 to 20 per cent by weight.

11. Agent according to one of Claims 6 to 10, **characterized in that** it additionally comprises at least one direct dye.

12. Agent according to one of Claims 6 to 11, **characterized in that** it has a pH of from 6.5 to 11.5.

13. Agent according to one of Claims 6 to 12, **characterized in that** it is present in the form of an aqueous or aqueous-alcoholic solution, a cream, a gel or an emulsion.

14. Agent according to one of Claims 6 to 13, **characterized in that** it is a hair colourant.

## Revendications

1. Dérivé de 1,4-diamino-2-(thiazol-2-yl)-benzène de formule (I) 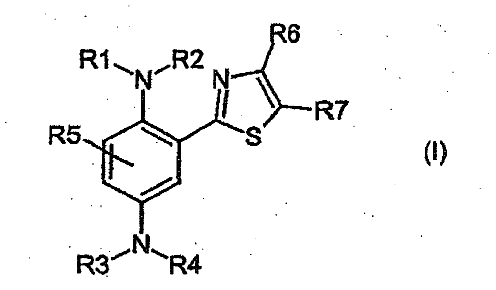 dans laquelle
**R1, R2, R3** et **R4** représentent, indépendamment les uns des autres, un atome d'hydrogène, un groupe alkyle en C₁-C₆, un groupe hydroxyalkyle en C₂-C₄, un groupe dihydroxyalkyle en C₃-C₄ ou un groupe alcoxy(C₁-C₄) -alkyle(C₁-C₂) ou bien R1 et R2 ou, respectivement, R3 et R4 forment un cycle aliphatique à 4-8 chaînons, au moins deux des radicaux R1 à R4 représentant un atome d'hydrogène ;
**R5** est un atome d'hydrogène ou d'halogène, un groupe alkyle en C₁-C₄, un groupe hydroxyalkyle en C₁-C₄ ou un groupe alcoxy en C₁-C₄ ;
**R6** et **R7** représentent, indépendamment l'un de l'autre, un atome d'hydrogène ou d'halogène, un groupe nitro, un groupe alcoxy en C₁-C₄, un groupe alkyle en C₁-C₆, un groupe hydroxyalkyle en C₁-C₄, un groupe dihydroxyalkyle en C₃-C₄ ou un radical de formule (II), 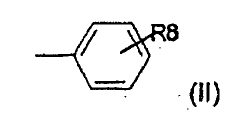 **R8** est un atome d'hydrogène, un groupe amino, un atome d'halogène, un groupe nitro ou un groupe hydroxy.

2. Dérivé de 1,4-diamino-2-(thiazol-2-yl)-benzène selon la revendication 1, **caractérisé en ce que**, dans la formule (I), un ou plusieurs des radicaux R5 à R7 représente(nt) un atome d'hydrogène.

3. Dérivé de 1,4-diamino-2-(thiazol-2-yl)-benzène selon la revendication 1 ou 2, **caractérisé en ce que**, dans la formule (I), les radicaux R1, R2, R3 et R4 sont des atomes d'hydrogène.

4. Dérivé de 1,4-diamino-2-(thiazol-2-yl)-benzène selon l'une quelconque des revendications 1 à 3, **caractérisé en ce que**, dans la formule (I), un ou les deux radicaux R6 et R7 représente(nt) un groupe alkyle en C₁-C₄ ou un groupe hydroxyalkyle en C₁-C₄.

5. Dérivé de 1,4-diamino-2-(thiazol-2-yl)-benzène selon l'une quelconque des revendications 1 à 4, **caractérisé en ce qu'**il est choisi parmi le 1,4-diamino-2-(thiazol-2-yl)-benzène, le 1,4-diamino-2-(4,5-diméthyl-thiazol-2-yl)-benzène, le 1,4-diamino-2-(4-phényl-thiazol-2-yl)-benzène et le 1,4-diamino-2-(4-méthyl-thiazol-2-yl)-benzène.

6. Composition pour la teinture par oxydation de fibres de kératine, à base d'une association développeur-coupleur, **caractérisée en ce qu'**elle contient en tant que développeur au moins un dérivé de 1,4-diamino-2-(thiazol-2-yl)-benzène de formule (I) selon l'une quelconque des revendications 1 à 5.

7. Composition selon la revendication 6, **caractérisée en ce qu'**elle contient le dérivé de 1,4-diamino-2-(thiazol-2-yl)-benzène de formule (I) en une quantité de 0,005 à 20,0 % en poids.

8. Composition selon la revendication 6 ou 7, **caractérisée en ce que** le coupleur est choisi parmi la 2,6-diaminopyridine, le 2-amino-4-[(2-hydroxy-éthyl)amino]anisole, le 2,4-diamino-1-fluoro-5-méthylbenzène, le 2,4-diamino-1-méthoxy-5-méthyl-benzène, le 2,4-diamino-1-éthoxy-5-méthylbenzène, le 2,4-diamino-1-(2-hydroxyéthoxy)-5-méthyl-benzène, le 2,4-di[(2-hydroxyéthyl)amino]-1,5-diméthoxybenzène, la 2,3-diamino-6-méthoxy-pyridine, la 3-amino-6-méthoxy-2-(méthylamino)-pyridine, la 2,6-diamino-3,5-diméthoxypyridine, la 3,5-diamino-2,6-diméthoxypyridine, le 1,3-diaminobenzène, le 2,4-diamino-1-(2-hydroxyéthoxy)-benzène, le 2,4-diamino-1,5-di(2-hydroxyéthoxy)-benzène, le 1-(2-aminoéthoxy)-2,4-diaminobenzène, le 2-amino-1-(2-hydroxyéthoxy)-4-méthylamino-benzène, l'acide 2,4-diaminophénoxyacétique, la 3-[di(2-hydroxyéthyl)amino]aniline, le 4-amino-2-di[(2-hydroxyéthyl)amino]-1-éthoxybenzène, le 5-méthyl-2-(1-méthyléthyl)phénol, la 3-[(2-hydroxyéthyl)amino]aniline, la 3-[(2-aminoéthyl)-amino]aniline, le 1,3-di(2,4-diaminophénoxy)-propane, le di(2,4-diaminophénoxy)méthane, le 1,3-diamino-2,4-diméthoxybenzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxy-phényl)amino]acétamide, le 5-[(2-hydroxyéthyl)-amino]-2-méthylphénol; le 3-[(2-hydroxyéthyl)-amino]phénol, le 3-[(2-méthoxyéthyl)amino]phénol, le 5-amino-2-éthylphénol, le 2-(4-amino-2-hydroxyphénoxy)éthanol, le 5-[(3-hydroxypropyl)-amino]-2-méthylphénol, le 3-[(2,3-dihydroxy-propyl)amino]-2-méthylphénol, le 3-[(2-hydroxyéthyl)amino]-2-méthylphénol, la 2-amino-3-hydroxypyridine, le 5-amino-4-chloro-2-méthylphénol, le 1-naphtol, le 1,5-dihydroxynaphtalène, le 1,7-dihydroxynaphtalène, le 2,3-dihydroxynaphtalène, le 2,7-dihydroxynaphtalène, l'acétate de 2-méthyl-1-naphtol, le 1,3-dihydroxybenzène, le 1-chloro-2,4-dihydroxybenzène, le 2-chloro-1,3-dihydroxybenzène, le 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxophénol, la 3,4-méthylènedioxyaniline, le 5-[(2-hydroxyéthyl)amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxy-indole, la 5,6-dihydroxy-indoline, le 5-hydroxy-indole, le 6-hydroxy-indole, le 7-hydroxy-indole et la 2,3-indolinedione.

9. Composition selon l'une quelconque des revendications 6 à 8, **caractérisée en ce que**, en plus du dérivé de 1,4-diamino-2-(thiazol-2-yl)-benzène de formule (I), elle contient au moins un autre développeur choisi dans le groupe constitué par le 1,4-diaminobenzène, le 2,5-diaminotoluène, l'alcool 2-(2,5-diaminophényl)éthylique, le 4-aminophénol et ses dérivés, des dérivés de 4,5-diaminopyrazole et les tétraaminopyrimidines.

10. Composition selon l'une quelconque des revendications 6 à 9, **caractérisée en ce que** les développeurs et les coupleurs sont contenus, respectivement, en une quantité totale de 0,005 à 20 % en poids, par rapport à la quantité totale de la composition de teinture par oxydation.

11. Composition selon l'une quelconque des revendications 6 à 10, **caractérisée en ce qu'**en outre elle contient au moins un colorant direct.

12. Composition selon l'une quelconque des revendications 6 à 11, **caractérisée en ce qu'**elle présente un pH de 6,5 à 11,5.

13. Composition selon l'une quelconque des revendications 6 à 12, **caractérisée en ce qu'**elle se trouve sous forme d'une solution aqueuse ou aqueuse-alcoolique, d'une crème, d'un gel ou d'une émulsion.

14. Composition selon l'une quelconque des revendications 6 à 13, **caractérisée en ce qu'**il s'agit d'une composition de teinture pour cheveux.
